# DEMANDE DE BREVET EUROPEEN

(11) **EP 1 666 084 A1**
(43) Date de publication de la demande: **07.06.2006**
(21) Numéro de dépôt: 04028411.9
(22) Date de dépôt: 01.12.2004
(51) Int. Cl.: A61M 37/00, A61M 5/32

(54) **Dispositif pour l'injection d'un principe actif pharmaceutique**

(71) Demandeur: Societe de Conseils de Recherches et d'Applications Scientifiques (S.C.R.A.S) SAS, 75016 Paris (FR)
(72) Inventeur: Aubert, Christophe, 1588 Cudrefin (CH); Cherif-Cheikh, Roland, 08860 Castelldefels, Barcelona (ES); Rimlinger, Thierry, 38080 L'Isle d'Abeau (FR); Bonacci, Fabrice, 69800 Saint Priest (FR); Barneaud, Serge, 06260 Puget Theniers (FR)
(74) Mandataire: Thérond, Gérard Raymond

(57) **Abrégé**

Dispositif d'injection pour l'injection d'un médicament solide (10) comprenant un corps (2) à l'intérieur duquel se déplace selon un axe d'avance général (X-X) une aiguille (4) à biseau (34) dans laquelle est introduit le médicament (10), ce dispositif d'injection (1) comprenant en outre des moyens de retenue pour prévenir la chute du médicament (10) avant injection, caractérisé en ce que ce la retenue du médicament (10) est réalisée par une déformation élastique imprimée à l'aiguille (4) par les moyens de retenue ou par une déformation élastique des moyens de retenue eux-mêmes, ou encore par une combinaison de la flexibilité de ces deux moyens.

## Description

La présente invention concerne un dispositif d'injection et, en particulier, un dispositif pour l'injection intramusculaire ou sous-cutanée d'un principe actif pharmaceutique.

Dans de nombreux cas, l'administration par voie parentérale de principes actifs pharmaceutiques peut être préférée à l'absorption par voie orale, notamment lorsque le médicament à administrer se dégrade partiellement ou totalement dans le système digestif ou lorsqu'on recherche une réponse rapide de l'organisme.

L'administration de principes médicamenteux par voie parentérale présente cependant certains inconvénients. L'un de ces inconvénients réside dans la gêne ressentie par le patient à qui le principe actif est administré. En effet, les préparations parentérales se présentent généralement sous la forme d'un grand volume de liquide dans lequel le médicament est en suspension ou dissous. Lorsque le médicament est faiblement soluble ou difficile à faire passer en suspension, ou bien encore lorsque le principe actif doit être administré à de fortes doses, on doit injecter un volume de liquide relativement important. Le rapport entre le principe actif et l'excipient est habituellement compris entre un pour cent et un pour mille. La gêne ressentie par le patient résulte ainsi tout à la fois de la taille de l'aiguille et du volume de liquide à injecter. Dans certains cas, la nature même de l'excipient peut également occasionner des souffrances au patient.

Un autre inconvénient de l'administration de médicaments dissous ou en suspension dans un média liquide réside dans le fait que les médicaments sont souvent instables dans le liquide. Le médicament et le liquide doivent donc être mélangés peu de temps avant l'injection. Ceci peut s'avérer particulièrement désavantageux lorsqu'il faut, par exemple, traiter des centaines de personnes dans un faible laps de temps pour enrayer une épidémie (administration d'un vaccin).

Pour pallier les inconvénients susmentionnés, on a eu recours à des médicaments sous forme solide plutôt que liquide pour mettre au point des préparations à libération prolongée ou contrôlée. La préparation se présente sous la forme d'un implant ou d'une tige qui est directement injecté au moyen d'un trocart. Un tel implant doit renfermer la dose quotidienne de médicament multipliée par le nombre de jours d'activité, et la quantité de média suffisante pour contrôler la vitesse de libération du médicament pour la période de temps considérée. Par conséquent, ces préparations solides à injecter nécessitent une aiguille bien plus grande que les aiguilles ordinairement utilisées avec les seringues, ce qui conduit à des injections douloureuses.

Le dispositif d'injection à sécurité décrit dans le brevet européen EP 0 783 342 remédie à ce dernier inconvénient. Il sera brièvement décrit en liaison avec la figure 1 annexée à la présente demande de brevet qui est une vue en coupe d'un tel dispositif d'injection dans sa position de repos.

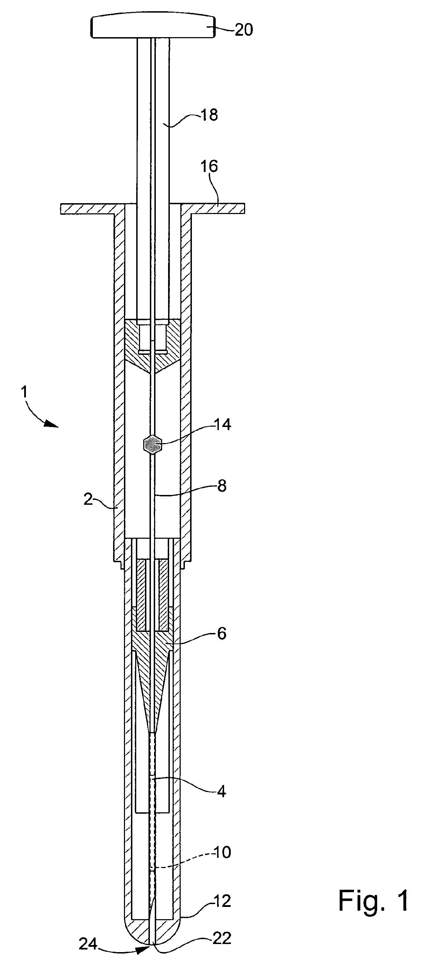

Désigné dans son ensemble par la référence numérique générale 1, le dispositif d'injection représenté sur la figure 1 comprend un corps 2 qui est fixé à une aiguille 4 par le biais de moyens de couplage 6. Une tige 8 est guidée en translation à l'intérieur de l'aiguille 4 et vient en butée contre une dose 10 de substance médicamenteuse disposée à l'intérieur de ladite aiguille 4. Une gaine creuse 12 entoure l'aiguille 4 de façon que celle-ci ne soit pas exposée avant utilisation. La tige 8 comporte un renflement 14 qui permet de limiter son parcours dans le corps 2. Le corps 2 comporte une collerette 16 pour faciliter le retrait du dispositif 1 après injection. Un piston 18 est fixé à l'extrémité proximale de la tige 8 et est agencé pour coulisser dans une extrémité proximale du corps 2. Il comporte une collerette 20. La gaine creuse 12 est placée de façon à coulisser à l'extrémité distale du corps 2 pour enfermer l'aiguille 4 lorsqu'elle est en position sortie.

Le fonctionnement du dispositif d'injection 1 brièvement décrit ci-dessus est le suivant. Lorsque ce dispositif est pressé contre la peau du patient, la gaine 12 coulisse sur le corps 2, exposant ainsi l'aiguille 4 et lui permettant de pénétrer dans la peau, tandis que le piston 18 et la tige 8, agencés à coulissement, maintiennent le médicament sous la peau lorsque l'aiguille 4 est retirée.

Le médicament solide à injecter est destiné à être immédiatement assimilé par le cops. Ainsi, les quantités injectées étant seulement celles nécessaires à l'obtention d'un effet immédiat, l'aiguille peut être aussi fine que celles de seringues classiques. L'injection est moins douloureuse dans la mesure où le volume injecté est considérablement moindre que le volume nécessaire à une injection sous forme liquide. D'autre part, l'aiguille du dispositif d'injection n'est pas exposée aux éléments extérieurs. De la sorte, l'aiguille ne pourra pas collecter de contaminants présents dans l'atmosphère ni érafler quelqu'un par inadvertance. De même, il ne sera pas possible d'injecter par inadvertance une fraction du médicament ou le sang du patient à un membre du personnel hospitalier.

Un joint 22 peut obturer l'ouverture 24 par laquelle l'aiguille 4 émerge du dispositif d'injection 1 afin de préserver la stérilité de ladite aiguille 4 et de la dose médicamenteuse 10. Ce joint 22 peut être réalisé en une matière friable telle que de la cire biocompatible et biodégradable. Alternativement, l'obturation de l'ouverture 24 peut être réalisée à l'aide d'un capuchon recouvrant complètement le manchon 12.

Ces moyens d'obturation du dispositif d'injection 1 ne sont pas satisfaisants. Dans le cas d'un bouchon de cire, comme il existe un risque non négligeable qu'un peu de cette matière reste accrochée à l'aiguille et soit injectée sous la peau du patient, le fabricant doit démontrer l'absence d'interaction entre la cire et le médicament injecté. Dans le cas d'un capuchon, on risque que l'implant tombe au moment où l'on retire ledit capuchon.

Il existait donc dans l'état de la technique un besoin pour un moyen permettant d'éviter la chute de l'implant avant utilisation du dispositif d'injection, notamment durant les périodes de stockage et lors de la manipulation dudit dispositif d'injection avant de procéder à l'injection proprement dite.

La présente invention a pour but de répondre à ce besoin ainsi qu'à d'autres encore en procurant un dispositif d'injection pour l'injection d'un médicament solide comprenant un corps à l'intérieur duquel se déplace une aiguille à biseau dans laquelle est introduit le médicament, ce dispositif d'injection comprenant en outre des moyens de retenue pour prévenir la chute du médicament avant injection, caractérisé en ce que la retenue du médicament est réalisée par une déformation élastique imprimée à l'aiguille par les moyens de retenue ou par une déformation élastique des moyens de retenue eux-mêmes, ou encore par une combinaison de la flexibilité de ces deux moyens.

Grâce à ces caractéristiques, la présente invention procure un dispositif d'injection d'un médicament solide encore appelé implant que l'on peut manipuler sans précaution exagérée tout en étant assuré que l'implant ne risque pas de tomber. En particulier, si l'extrémité ouverte du dispositif d'injection par laquelle sort l'aiguille est obturée par un capuchon, on peut, au moment où l'on s'apprête à procéder à l'injection, retirer ce capuchon sans craindre que l'implant ne tombe. En outre, comme on joue sur l'élasticité de l'aiguille et/ou des moyens de retenue, on n'est pas obligé de se livrer à des manipulations compliquées pour retirer lesdits moyens de retenue avant de pouvoir utiliser ledit dispositif. Par ailleurs, les moyens de retenue ne gênent en rien le bon fonctionnement du dispositif d'injection.

Selon une caractéristique complémentaire de l'invention, les moyens de retenue obturent temporairement le biseau de l'aiguille en position de repos, empêchant ainsi l'implant de sortir de l'aiguille et de tomber.

Selon un premier mode de réalisation de l'invention, les moyens de retenue permettent d'excentrer l'ouverture par laquelle l'aiguille sort par rapport à l'axe d'avance général de ladite aiguille à l'intérieur du dispositif d'injection.

Selon un second mode de réalisation, les moyens de retenue comprennent une languette élastique qui vient temporairement en appui contre le biseau de l'aiguille.

D'autres caractéristiques et avantages de la présente invention ressortiront plus clairement de la description détaillée qui suit de deux modes de réalisation du dispositif d'injection selon l'invention, ces exemples étant donnés à titre purement illustratif et non limitatif seulement, en liaison avec le dessin annexé sur lequel :
- la figure 1, déjà mentionnée, est une vue en coupe d'un dispositif d'injection d'un médicament solide;
- les figures 2A et 2B sont des vues en coupe de l'extrémité distale d'un dispositif d'injection muni de moyens permettant d'éviter la chute du médicament selon une première forme d'exécution de l'invention, l'aiguille étant respectivement en position de repos et en position sortie;
- les figures 3A et 3B sont des vues en coupe de l'extrémité distale d'un dispositif d'injection muni de moyens permettant d'éviter la chute du médicament selon une seconde forme d'exécution de l'invention, l'aiguille étant respectivement en position de repos et en position sortie, et
- les figures 4A et 4B sont des vues en coupe de l'extrémité distale d'un dispositif d'injection muni de moyens permettant d'éviter la chute du médicament selon une troisième forme d'exécution de l'invention, l'aiguille étant respectivement en position de repos et en position sortie.

La présente invention procède de l'idée générale inventive qui consiste à munir un dispositif d'injection d'un médicament solide encore appelé implant de moyens permettant de prévenir la chute du médicament lors des périodes de stockage ou juste avant de procéder à l'injection. En jouant sur l'élasticité de l'aiguille ou sur leur propre élasticité, ces moyens n'obligent pas l'utilisateur à se livrer à des manipulations compliquées pour rendre ledit dispositif opérationnel. Par ailleurs, garantissant que l'implant ne tombera pas en position de repos de l'aiguille, les moyens de retenue selon l'invention ne gênent en rien la sortie de l'aiguille et le bon fonctionnement général du dispositif d'injection.

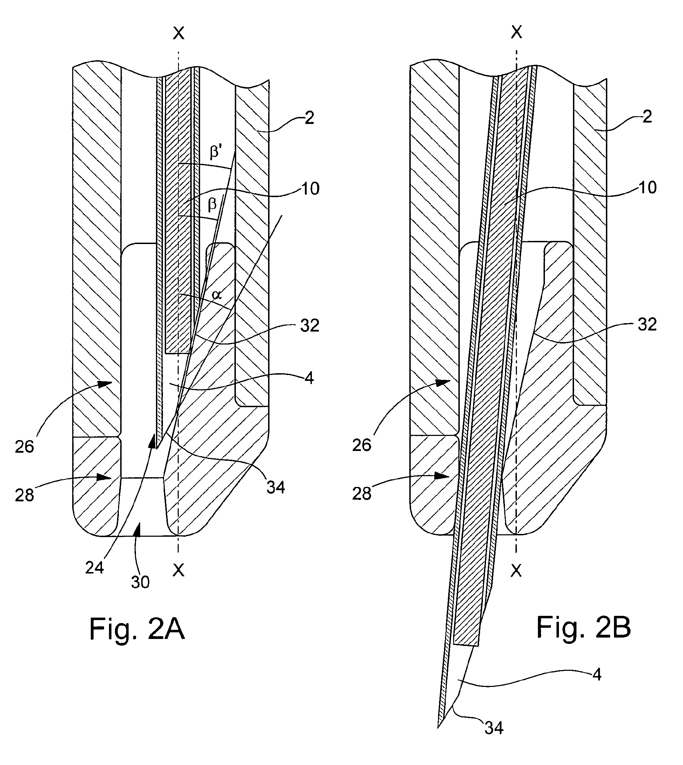

Une première forme de réalisation des moyens de retenue selon l'invention est illustrée aux figures 2A et 2B. Dans tout ce qui suit, les éléments déjà décrits en liaison avec la figure 1 seront désignés par les mêmes références numériques. Le dispositif d'injection 1 décrit en liaison avec la figure 1 est un exemple donné à titre purement illustratif et non limitatif du type de dispositif d'injection auquel la présente invention peut s'appliquer.

L'extrémité distale 26 du corps 2 du dispositif d'injection est munie d'un insert 28. Cet insert 28 est réalisé en un matériau plastique biocompatible et stérilisable par exemple par irradiation tel qu'un polycarbonate. Il est introduit à l'intérieur du corps 2 par l'ouverture 24 par laquelle l'aiguille 4 sort. L'insert 28, de forme générale cylindrique, a un diamètre extérieur adapté au diamètre intérieur du corps 2, de façon que les forces de frottement entre ces deux pièces soient suffisantes pour éviter tout risque de chute intempestif dudit insert 28.

Comme on peut le voir à l'examen des figures, l'insert 28 comprend à sa base une ouverture 30 par laquelle sort l'aiguille 4. Cette ouverture 30 est excentrée par rapport à l'axe général d'avance X-X de l'aiguille 4. A cet effet, l'insert 28 présente, sur sa face en regard de ladite aiguille 4, un plan incliné 32 qui, du haut vers le bas, se rapproche de l'axe d'avance de cette aiguille 4 jusqu'à venir l'intercepter et conduit jusqu'à l'ouverture 30. Ainsi, en position de repos (figure 2A), l'aiguille 4 est en appui par son ouverture biseautée 34 contre le plan incliné 32, ce qui empêche la dose 10 de substance médicamenteuse de tomber. Lors du mouvement de sortie de l'aiguille 4 (figure 2B), celle-ci glisse sur le plan incliné 32 en se déformant. Ce mouvement est rendu possible grâce à la flexibilité de l'aiguille 4. L'ouverture biseautée 34 qui constitue la partie tranchante de l'aiguille 4 est inclinée d'un angle α par rapport à l'axe X-X. Cette ouverture biseautée 34 est précédée par une surface de glissement 36 inclinée par rapport à l'axe X-X d'un angle β inférieur à l'angle α par laquelle ladite aiguille 4 glisse sur le plan incliné 32. Avantageusement, le plan incliné 32 de l'insert 28 est incliné par rapport à l'axe X-X d'un angle β' sensiblement égal à l'angle β. De la sorte, on évite le contact entre la partie tranchante de l'aiguille 4 et le plan incliné 32 de l'insert 28 qui pourrait conduire à l'arrachement d'un bout de plastique.

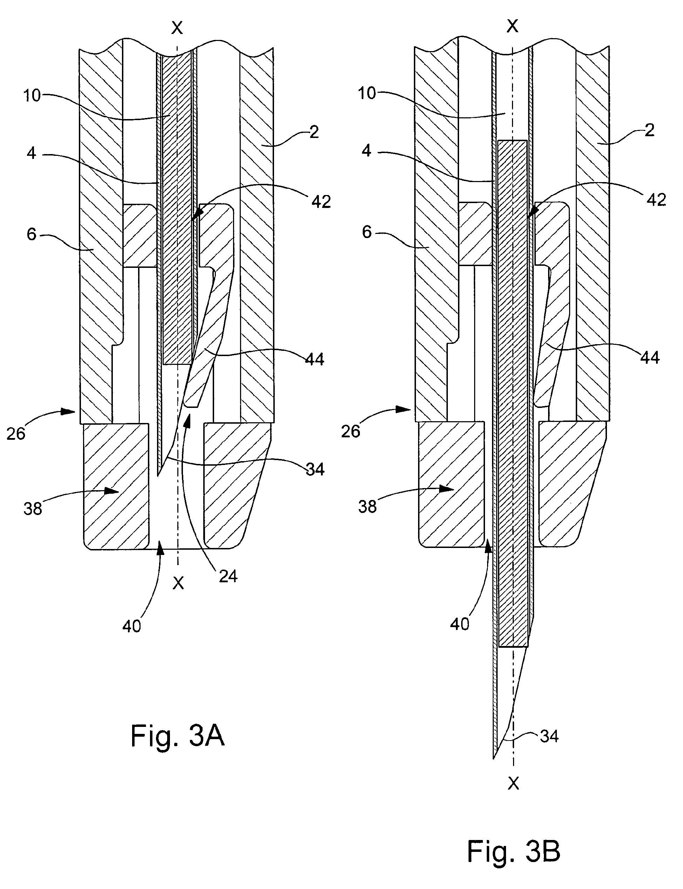

Une seconde forme de réalisation des moyens de retenue selon l'invention est représentée aux figures 3A et 3B. L'extrémité distale 26 du corps 2 du dispositif d'injection est munie d'un insert 38. Comme dans le cas ci-dessus, l'insert 38 est réalisé par exemple en un matériau plastique tel que du polycarbonate et est introduit à l'intérieur du corps 2 par l'ouverture 24 par laquelle sort l'aiguille 4. L'insert 38 est fermement retenu à l'intérieur du corps 2 par le biais des forces de frottement entre ledit insert 38 et la paroi intérieure dudit corps 2.

L'insert 38 présente deux ouvertures traversantes 40 et 42 alignées sur l'axe général d'avance X-X de l'aiguille 4 à l'intérieur du corps 2. La première de ces ouvertures 40 permet la sortie de l'aiguille 4 hors du corps 2 du dispositif d'injection au moment de procéder à l'injection (voir figure 3B). La seconde ouverture 42 permet d'enfiler l'insert 38 sur l'aiguille 4 au moment du montage dudit insert 38. Au cours du mouvement d'introduction axiale de l'insert 38 à l'intérieur du corps 2, l'aiguille 4 vient écarter une languette élastique 44 de sa position de repos. Sous l'effet de la force de rappel élastique, la languette 44 vient en appui contre la face en biseau 34 de ladite aiguille 4, empêchant la dose 10 de substance médicamenteuse de tomber. Lors de l'injection, l'aiguille 4 avance relativement à l'insert 38 et repousse la languette élastique 44 (voir figure 3B).

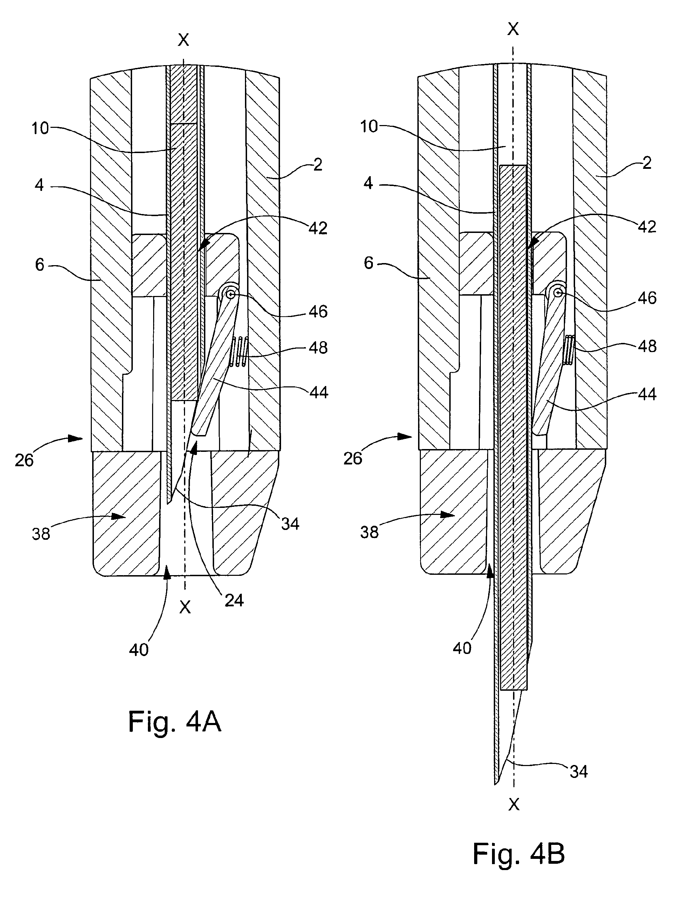

Les figures 4A et 4B sont des vues en coupe d'une variante de réalisation de l'insert 38 décrit ci-dessus. Au lieu de venir de matière avec l'insert 38, la languette élastique 44 est articulée à pivotement sur ledit insert 38 par le biais d'une charnière 46 et est maintenue en appui contre la face en biseau 34 de l'aiguille 4 sous l'effet de la force de rappel élastique d'un ressort 48. Lors de l'avance de l'aiguille 4, la languette 44 s'efface, ce qui provoque la compression du ressort 48.

Il va de soi que la présente invention n'est pas limitée aux modes de réalisation qui viennent d'être décrits et que diverses modifications et variantes simples peuvent être envisagées sans sortir du cadre de l'invention tel que défini par les revendications annexées.

## Revendications

1. Dispositif d'injection pour l'injection d'un médicament solide (10) comprenant un corps (2) à l'intérieur duquel se déplace selon un axe d'avance général (X-X) une aiguille (4) à biseau (34) dans laquelle est introduit le médicament (10), ce dispositif d'injection (1) comprenant en outre des moyens de retenue pour prévenir la chute du médicament (10) avant injection, **caractérisé en ce que** ce la retenue du médicament (10) est réalisée par une déformation élastique imprimée à l'aiguille (4) par les moyens de retenue ou par une déformation élastique des moyens de retenue eux-mêmes, ou encore par une combinaison de la flexibilité de ces deux moyens.

2. Dispositif d'injection selon la revendication 1, **caractérisé en ce que** les moyens de retenue obturent temporairement le biseau (34) de l'aiguille (4), empêchant ainsi le médicament (10) de sortir de l'aiguille (4) et de tomber.

3. Dispositif d'injection selon la revendication 2, **caractérisé en ce que** les moyens de retenue comprennent une ouverture (30) par laquelle sort l'aiguille (4) et qui est excentrée par rapport à l'axe général d'avance (X-X) de ladite aiguille (4) à l'intérieur du corps (2).

4. Dispositif d'injection selon la revendication 3, **caractérisé en ce que** les moyens de retenue présentent, en regard du biseau (34) de l'aiguille (4), un plan incliné (32) qui se rapproche de l'axe d'avance (X-X) de cette aiguille (4) jusqu'à venir l'intercepter et qui conduit jusqu'à l'ouverture (30).

5. Dispositif d'injection selon la revendication 4, **caractérisé en ce que** l'aiguille présente une surface de glissement (36) dont l'inclinaison est sensiblement égale à celle du plan incliné (32).

6. Dispositif d'injection selon la revendication 2, **caractérisé en ce que** les moyens de retenue comprennent une languette élastique (44) qui vient temporairement en appui contre le biseau (34) de l'aiguille (4).

7. Dispositif d'injection selon la revendication 6, **caractérisé en ce que**, en sortant, l'aiguille (4) repousse la languette élastique (44).

8. Dispositif selon l'une quelconque des revendications 6 ou 7, **caractérisé en ce que** la languette élastique (44) vient de matière avec les moyens de retenue.

9. Dispositif d'injection selon l'une quelconque des revendications 6 ou 7, **caractérisé en ce que** la languette élastique (44) est articulée à pivotement sur les moyens de retenue par le biais d'une charnière (46) et est maintenue en appui contre le biseau (34) de l'aiguille (4) sous l'effet de la force de rappel élastique d'un ressort (48).

10. Dispositif d'injection selon l'une quelconque des revendications 6 à 9, **caractérisé en ce que** les moyens de retenue présentent une première ouverture traversante (40) qui permet la sortie de l'aiguille (4), et une seconde ouverture traversante (42) par laquelle lesdits moyens de retenue sont enfilés sur ladite aiguille (4), ces deux ouvertures (40, 42) étant alignées sur l'axe général d'avance (X-X) de l'aiguille (4) à l'intérieur du corps (2).

11. Dispositif d'injection selon l'une quelconque des revendications 1 à 10, **caractérisé en ce que** les moyens de retenue se présentent sous la forme d'un insert (28; 38).

12. Dispositif d'injection selon la revendication 11, **caractérisé en ce que** l'insert (28; 38) est réalisé en un matériau biocompatible et stérilisable.

13. Dispositif d'injection selon la revendication 12, **caractérisé en ce que** l'insert (28; 38) est réalisé en un matériau plastique.

14. Dispositif d'injection selon la revendication 13, **caractérisé en ce que** l'insert (28; 38) est réalisé en polycarbonate.
